# EUROPEAN PATENT SPECIFICATION

(11) **EP 2 334 396 B1**
(45) Date of publication and mention of the grant of the patent: **13.11.2013**
(21) Application number: 09791760.3
(22) Date of filing: 20.08.2009
(51) Int. Cl.: B01D 11/04, A61K 9/16

(54) **METHODS OF PROCESSING MICROPARTICLES**
VERFAHREN ZUR VERARBEITUNG VON MIKROTEILCHEN
PROCÉDÉS DE TRAITEMENT DE MICROPARTICULES

(30) Priority: 20.08.2008 US 195182
(43) Date of publication of application: 22.06.2011
(73) Proprietor: Baxter International Inc., Deerfield, IL 60015 (US); Baxter Healthcare S.A., 8152 Glattpark (Opfikon) (CH)
(72) Inventor: TSUNG, Mei, Wellesley MA 02482 (US); EASSON, D., Davidson, Shrewsbury MA 01545 (US); MEHR, Eugene, Belmont MA 02478 (US); BOURHIS, Alain, L., Natick MA 01760 (US)
(74) Representative: Potter Clarkson LLP
(86) International application number: PCT/US2009/054534
(87) International publication number: WO 2010/022282

(56) References cited:
- WO-A1-2004/062784
- US-A- 4 732 333
- US-A- 6 090 925
- US-A- 6 113 795
- US-A1- 2002 179 540
- US-A1- 2003 075 817
- US-A1- 2004 200 774
- US-A1- 2005 170 005
- US-B2- 6 998 051

## Description

### BACKGROUND OF THE INVENTION

### Technical Field

The present disclosure relates to microparticles, compositions and formulations containing microparticles, and more specifically, to methods for processing such compositions and formulations.

### Description of Related Technology

Microparticles, microspheres, and microcapsules, referred to herein collectively as "microparticles," are solid or semi-solid particles having a diameter of less than one millimeter, more preferably less than 100 microns, which can be formed of a variety of materials, including but not limited to various polymers and proteins. Microparticles have been used in many different applications, primarily separations, diagnostics, and drug delivery.

The most well known examples of microparticles used in separations techniques are those which are formed of polymers of either synthetic or protein origin, such as polyacrylamide, hydroxyapatite, or agarose. These polymeric microparticles are commonly used to separate molecules such as proteins based on molecular weight and/or ionic charge, or by interaction with molecules chemically coupled to the microparticles.

In the diagnostic area, spherical beads or particles have been commercially available as a tool for biochemists for many years. For example, microparticles have been derivatized with an enzyme, a substrate for an enzyme, or a labeled antibody, and then interacted with a molecule to be detected, either directly or indirectly. A number of derivatized beads are commercially available with various constituents and sizes.

In the controlled drug delivery area, molecules have been encapsulated within microparticles or incorporated into a matrix to provide controlled release of the molecules. A number of different techniques have been used to make such microparticles from various polymers including phase separation, solvent evaporation, emulsification, and spray drying. Generally, the polymers form the supporting structure of the microparticles, and the drug or molecule of interest is incorporated into the supporting structure. Exemplary polymers used for the formation of microparticles include homopolymers and copolymers of lactic acid and glycolic acid (PLGA), block copolymers, and polyphosphazenes.

U.S. Patent Publication No. 2005/0170005 (the '005 publication) discloses phase separation methods for forming microparticles involving dissolving an active agent in an aqueous and/or aqueous-miscible solvent(s) containing a dissolved phase-separation enhancing agent(s) to form a solution in a single liquid phase. The solution is then subjected to a liquid-solid phase separation to cause the active agent to form solid spherical small particles (i.e., the solid phase) while the phase-separation enhancing agent(s) and solvent(s) comprise the liquid phase. The '005 publication discloses methods of harvesting microparticles including washing solutions comprising microparticles and/or concentrated microparticles with liquid media in which the active agent is insoluble and the undesired phase-separation enhancing agent is soluble. Disclosed liquid media include organic solvents and supercritical fluids. Representative washing methods include diafiltration and centrifugation. The remaining liquid phases are then typically removed by lyophilization or evaporation.

US-4,732,333 discloses a separation device comprising a container having an aperture in its bottom, a filter element mounted at the bottom of the container for covering the aperture, and a rotary blade device for preventing clogging of the filter and/or for stirring.

### SUMMARY OF THE INVENTION

According to the present invention there is provided a method for processing multi-phasic dispersions according to claim 1.

In one embodiment, the methods for processing multi-phasic dispersions involve pressurizing the chamber with a first gas to a pressure greater than 10 bar but less than the supercritical pressure of the gas.

In an embodiment, the methods for processing multi-phasic dispersions comprise providing a multi-phasic dispersion including dispersed and continuous phases, the dispersion comprising solid microparticles and at least a non-volatile material and optionally a solvent, positioning the multi-phasic dispersion within an extraction basket, positioning the extraction basket within a chamber capable of being extraction basket, positioning the extraction basket within a chamber capable of being pressurized, pressurizing the chamber with a first gas to a pressure less than the supercritical pressure of the gas, contacting the multi-phasic dispersion with the first gas, thereby causing at least a portion of the non-volatile material and optionally the solvent to flow through the extraction basket, pressurizing the chamber with a second gas to a pressure greater than or equal to the supercritical pressure of the second gas and heating the second gas, thereby providing a supercritical fluid or a sub-critical fluid within the chamber, and contacting the multi-phasic dispersion with the fluid, thereby separating residual non-volatile material and optionally solvent from the multi-phasic dispersion to provide microparticles that are substantially free of the non-volatile material and optionally the solvent.

In yet another embodiment, the methods for processing multi-phasic dispersions involve spraying a combination of (i) a supercritical fluid or a sub-critical fluid and (ii) a multi-phasic dispersion into an extraction basket of a chamber capable of being pressurized, thereby separating at least a portion of at least a non-volatile material and optionally a solvent from solid microparticles, wherein the multi-phasic dispersion includes dispersed and continuous phases and comprises the solid microparticles and at least the non-volatile material and optionally the solvent.

### BRIEF DESCRIPTION OF THE DRAWINGS

Exemplary aspects and features in accordance with the disclosure are described and explained in greater detail below with the aid of the sole drawing figure in which:

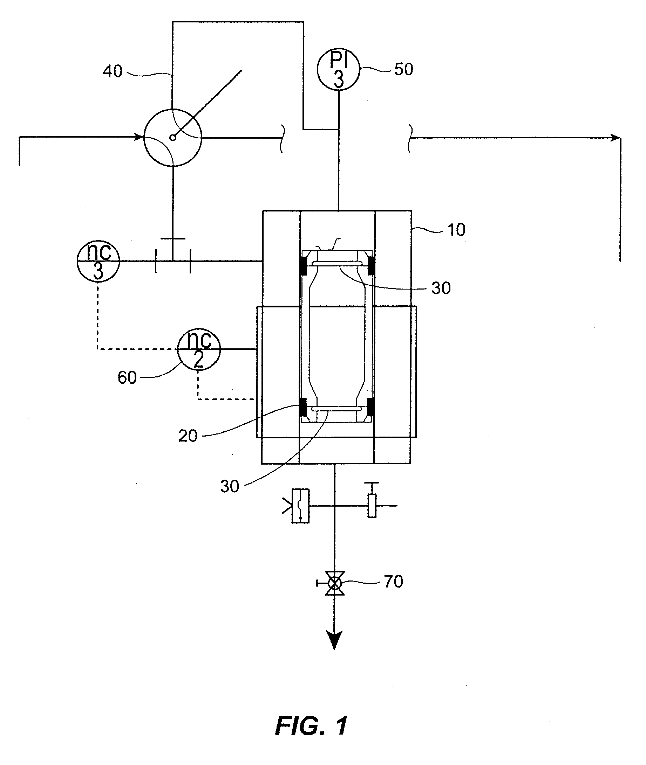

Figure 1 illustrates a suitable apparatus for carrying out the methods in accordance with the disclosure.

### DETAILED DESCRIPTION

The present disclosure relates to compositions and formulations containing microparticles and to methods for processing such compositions and formulations. In accordance with the methods, microparticles can be separated from reaction/incubation media so that the microparticles can be collected and/or incorporated into compositions and formulations suitable for drug delivery, diagnostic, separations, and other applications.

The disclosed methods are advantageous for a number of reasons including but not limited to (1) multi-phasic dispersions can be processed so that various components typically contained within the continuous phase are removed while the discontinuous or dispersed phase (e.g., microparticles) is harvested in a single process; (2) organic solvents are not required to remove polymer(s), salt(s), solvent(s), and/or excipient(s) from the microparticles; (3) additional expensive and time-consuming drying steps such as lyophilization and evaporation (e.g., under reduced pressure and elevated temperature) are not necessary to remove residual solvent or lyophilizable solutes from the microparticles; and (4) concentration steps such as centrifugation and diafiltration can optionally be avoided. Beneficially, the obtained microparticles can be substantially free of residual polymer(s), salt(s), solvent(s), and/or excipient(s) added during microparticle formation. Accordingly, the disclosed methods facilitate production of microparticles comprising substantially 100 wt.% active agent(s) and/or macromolecule carrier(s), which are substantially free of residual polymer(s), salt(s), solvent(s), and/or excipient(s).

Unless otherwise defined herein, scientific and technical terminologies employed in the present disclosure shall have the meanings that are commonly understood and used by one of ordinary skill in the art. Unless otherwise required by context, it will be understood that singular terms shall include plural forms of the same and plural terms shall include the singular. Specifically, as used herein and in the claims, the singular forms "a" and "an" include the plural reference unless the context clearly indicates otherwise. Thus, for example, the reference to a microparticle is a reference to one such microparticle or a plurality of such microparticles, including equivalents thereof known to one skilled in the art. Also, as used herein and in the claims, the terms "at least one" and "one or more" have the same meaning and include one, two, three or more. The following terms, unless otherwise indicated, shall be understood to have the following meanings when used in the context of the present disclosure.

"Dispersion" refers to a mixture of matters having at least one dispersed or discontinuous phase (optionally, being finely divided, such as in the form of solid microparticles) present in a solid or non-solid continuous phase (e.g., fluidic, liquid, aqueous, organic, gaseous). Representative examples of dispersions in accordance with the disclosure include solid in solid, solid in liquid, solid in gas, and the like. A dispersion can be substantially homogenous or non-homogenous. A suspension is a particular dispersion in which the dispersed solid phase (such as microparticles) can remain stably suspended (substantially free of sedimentation) in the continuous phase for extended periods of time (for example, at least 5 seconds, 10 seconds, or 30 seconds, e.g., minutes, hours, days, weeks, months, or even one year or more). "Multi-phasic dispersions" are dispersions having at least two phases, for example, three or even more phases. In one example, such dispersions may comprise two immiscible fluids (e.g., solvents or solvent systems optionally containing other ingredients dissolved or dispersed therein) in addition to a dispersed phase.

"Microparticle" refers to a solid particulate (including substantially solid or semi-solid, but excluding gel, liquid and gas) having an average geometric particle size (sometimes referred to as diameter) of less than about 1 mm, for example, less than about 200 microns, less than about 100 microns, less than about 10 microns, less than about 1 micron, less than about 100 nm, less than about 10 nm, greater than about 0.1 nm, greater than about 1 nm, and ranges between these values. Thus, suitable ranges for average geometric particle size include about 0.1 nm to about 1 mm, about 1 nm to about 1 mm, about 10 nm to about 1 mm, about 100 nm to about 1 mm, about 1 micron to about 1 mm, about 10 microns to about 1 mm, about 100 microns to about 1 mm, about 200 microns to about 1 mm, about 0.1 nm to about 200 microns, about 1 nm to about 200 microns, about 10 nm to about 200 microns, about 100 nm to about 200 microns, about 1 micron to about 200 microns, about 10 microns to about 200 microns, about 100 microns to about 200 microns, about 0.1 nm to about 100 microns, about 1 nm to about 100 microns, about 10 nm to about 100 microns, about 100 nm to about 100 microns, about 1 micron to about 100 microns, about 10 microns to about 100 microns, about 0.1 nm to about 10 microns, about 1 nm to about 10 microns, about 10 nm to about 10 microns, about 100 nm to about 10 microns, about 1 micron to about 10 microns, about 0.1 nm to about 1 micron, about 1 nm to about 1 micron, about 10 nm to about 1 micron, about 100 nm to about 1 micron, about 0.1 nm to about 100 nm, about 1 nm to about 100 nm, about 10 nm to about 100 nm, about 0.1 nm to about 10 nm, about 1 nm to about 10 nm, and/or about 0.1 nm to about 1 nm. Average geometric particle size can be measured by dynamic light scattering methods (such as photocorrelation spectroscopy, laser diffraction, low-angle laser light scattering (LALLS), medium-angle laser light scattering (MALLS)), light obscuration methods (such as Coulter analysis method), or other methods (such as rheology, light or electron microscopy). Microparticles for pulmonary delivery have an aerodynamic particle size as determined by time of flight measurements or Andersen Cascade Impactor measurements. Microparticles having a spherical shape are sometimes referred to as microspheres and nanospheres. Microparticles having an encapsulated structure are sometimes referred to as microcapsules and nanocapsules. Microparticles can be porous, for example, having one or more internal voids and/or cavities. Other microparticles are non-porous and/or are free of such voids or cavities. Microparticles are formed from, in part or in whole, one or more materials including but not limited to active agents, carriers, polymers, complexing agents, stabilizing agents, excipients, ions, moisture, residual solvents, impurities, by-products, and/or manufacturing-related compounds. Microparticles can be crystalline, amorphous, microcrystalline, nanocrystalline, or a combination thereof.

"Active agent" refers to naturally occurring, recombinant, synthetic, or semi-synthetic materials (e.g., compounds, fermentates, extracts, cellular structures) capable of eliciting, directly or indirectly, one or more physical, chemical, and/or biological effects, *in vitro* and/or *in vivo.* The active agent can be capable of preventing, alleviating, treating, and/or curing abnormal and/or pathological conditions of a living body, such as by destroying a parasitic organism, or by limiting the effect of a disease or abnormality by materially altering the physiology of the host or parasite. The active agent can be capable of maintaining, increasing, decreasing, limiting, or destroying a physiological body function. The active agent can be capable of diagnosing a physiological condition or state by an *in vitro* and/or *in vivo* test. The active agent can be capable of controlling or protecting an environment or living body by attracting, disabling, inhibiting, killing, modifying, repelling and/or retarding an animal or microorganism. The active agent can be capable of otherwise treating (such as deodorizing, protecting, adorning, grooming) a body. Depending on the effect and/or its application, the active agent can further be referred to as a bioactive agent, a pharmaceutical agent (such as a prophylactic agent, a therapeutic agent), a diagnostic agent, a nutritional supplement, and/or a cosmetic agent, and includes, without limitation, examples such as prodrugs, affinity molecules, synthetic organic molecules, polymers, molecules with a molecular weight of 2kD or less (such as those having a molecular weight of less than about 1.5kD, or less than about 1kD), macromolecules (such as those having a molecular weight of greater than about 2kD, for example, greater than about 5kD or between about 2kD and about 5kD), proteinaceous compounds, peptides, vitamins, steroids, steroid analogs, lipids, nucleic acids, carbohydrates, precursors thereof, and derivatives thereof. Active agents can be ionic or non-ionic, can be neutral, positively charged, negatively charged, or zwitterionic, and can be used singly or in combination of two or more thereof. Active agents can be water-insoluble or water-soluble. Active agents can have an isoelectric point of 7.0 or greater, or less than 7.0.

"Proteinaceous compounds" refer to natural, synthetic, semi-synthetic, or recombinant compounds of or related structurally and/or functionally to proteins, such as those containing or consisting essentially of α-amino acids covalently associated through peptide linkages. Non-limiting proteinaceous compounds include globular proteins (e.g., albumins, globulins, histones), fibrous proteins (e.g., collagens, elastins, keratins), compound proteins (including those containing one or more non-peptide components, e.g., glycoproteins, nucleoproteins, mucoproteins, lipoproteins, metalloproteins), therapeutic proteins, fusion proteins, receptors, antigens (such as synthetic or recombinant antigens), viral surface proteins, hormones and hormone analogs, antibodies (such as monoclonal or polyclonal antibodies), enzymes, Fab fragments, cyclic peptides, linear peptides, and the like. Non-limiting therapeutic proteins include bone morphogenic proteins, drug resistance proteins, toxoids, erythropoietins, proteins of the blood clotting cascade (e.g., Factor VII, Factor VIII, Factor IX, et al.), subtilisin, ovalbumin, alpha-1-antitrypsin (AAT), DNase, superoxide dismutase (SOD), lysozymes, ribonucleases, hyaluronidase, collagenase, human growth hormone (hGH), erythropoietin, insulin, insulin-like growth factors, interferons, glatiramer, granulocyte-macrophage colony-stimulating factor, granulocyte colony-stimulating factor, desmopressin, leutinizing hormone release hormone (LHRH) agonists (e.g., leuprolide, goserelin, buserelin, gonadorelin, histrelin, nafarelin, deslorelin, fertirelin, triptorelin), LHRH antagonists, vasopressin, cyclosporine, calcitonin, parathyroid hormone, parathyroid hormone peptides, glucogen-like peptides, and analogs thereof. Proteinaceous compounds may be neutral, positively charged, negatively charged, or zwitterionic, and may be used singly or in combination of two or more thereof.

"Nucleic acids" refer to natural, synthetic, semi-synthetic, or recombinant compounds formed at least in part from two or more of the same or different nucleotides, and may be single-stranded or double-stranded. Non-limiting examples of nucleic acids include oligonucleotides (such as those having 20 or less base pairs, e.g., sense, anti-sense, or missense), aptamers, polynucleotides (e.g., sense, anti-sense, or missense), DNA (e.g., sense, anti-sense, or missense), RNA (e.g., sense, anti-sense, or missense), siRNA, nucleotide acid constructs, single-stranded or double-stranded segments thereof, as well as precursors and derivatives thereof (e.g., glycosylated, hyperglycosylated, PEGylated, FITC-labeled, nucleosides, salts thereof). Nucleic acids may be neutral, positively charged, negatively charged, or zwitterionic, and may be used singly or in combination of two or more thereof.

"Macromolecule" refers to a material capable of providing a three-dimensional (e.g., tertiary and/or quaternary) structure, and includes carriers and certain active agents of the present disclosure. Macromolecules typically have a molecular weight of 2 kD or greater, for example, greater than 5kD or from 2kD to 5kD. Non-limiting macromolecules used to form the microparticles include, *inter alia,* polymers, copolymers, proteins (e.g., enzymes, recombinant proteins, albumins such as human serum albumin, monoclonal antibodies, polyclonal antibodies, proteinaceous compounds), peptides, lipids, carbohydrates (e.g., monosaccharides, disaccharides, polysaccharides), nucleic acids, vectors (e.g., viruses, viral particles), and complexes and conjugates thereof (e.g., covalent and/or non-covalent associations between two macromolecules such as carbohydrate-protein complexes or conjugates, or between an active agent and a macromolecule such as hapten-protein complexes or conjugates). Macromolecules may be neutral, positively charged, negatively charged, or zwitterionic, and may be used singly or in combination of two or more thereof.

"Carrier" refers to a compound, typically a macromolecule, having a primary function to provide a three-dimensional structure (including tertiary and/or quaternary structure) to the microspheres. The carrier may be unassociated or associated with the active agent (such as conjugates or complexes thereof) in forming microparticles as described above. The carrier may further provide other functions, such as being an active agent, modifying a release profile of the active agent from the microparticle, and/or imparting one or more particular properties to the microparticle (such as contribute at least in part to the net surface charge). In one example, the carrier is a protein (e.g., an albumin such as human serum albumin) having a molecular weight of 1500 Daltons or greater.

"Polymer" or "polymeric" refers to a natural, recombinant, synthetic, or semi-synthetic molecule having in at least one main chain, branch, or ring structure two or more repeating monomer units. Polymers broadly include dimers, trimers, tetramers, oligomers, higher molecular weight polymers, adducts, homopolymers, random copolymers, pseudo-copolymers, statistical copolymers, alternating copolymers, periodic copolymers, bipolymers, terpolymers, quaterpolymers, other forms of copolymers, substituted derivatives thereof, and mixtures thereof. In one aspect, the terms polymer and polymeric refer to molecules having 10 or more repeating monomer units. Polymers can be linear, branched, block, graft, monodisperse, polydisperse, regular, irregular, tactic, isotactic, syndiotactic, stereoregular, atactic, stereoblock, single-strand, double-strand, star, comb, dendritic, and/or ionomeric, can be ionic or non-ionic, can be neutral, positively charged, negatively charged, or zwitterionic, and can be used singly or in combination of two or more thereof.

"Spherical" refers to a geometric shape that is at least "substantially spherical." "Substantially spherical" means that the ratio of the longest length (i.e., one between two points on the perimeter and passes the geometric center of the shape) to the shortest length on any cross-section that passes through the geometric center is less than about 1.5, such as less than about 1.33, or less than about 1.25. Thus, spherical does not require a line of symmetry. Further, the microparticles can have surface texturing (such as continuous or discrete lines, islands, lattice, indentations, channel openings, protuberances that are small in scale when compared to the overall size of the microparticles) and still be considered spherical. Surface contact between microparticles is minimized when the microparticles are spherical, and thus undesirable agglomeration of the microparticles is typically minimized. In comparison, microparticles that are aspherical crystals or flakes typically display observable agglomeration through ionic and/or non-ionic interactions at relatively large flat surfaces.

"Solid" refers to a state that includes at least substantially solid and/or semi-solid, but excludes liquid and gas.

"Ambient temperature" refers to a temperature of around room temperature, typically in a range of about 20°C to about 40°C, for example, about 20°C to about 25°C.

"Formed from" and "formed of" denote open language. As such, it is intended that a composition formed from or formed of a list of recited components be a composition comprising at least these recited components, and can further include other non-recited components during formulation of the composition and/or in the final obtained product.

"Supercritical fluid" refers to a fluid having a pressure exceeding its critical pressure and a temperature exceeding its critical temperature (i.e., both the temperature and the pressure are above the critical point of the fluid).

"Sub-critical fluid" refers to a fluid or condensed phase having a sub-critical temperature ratio between about 0.95 and about 1.0, wherein the sub-critical temperature ratio equals the actual temperature divided by the critical temperature (TR_{sub-critical} = T_{actual}/T_{c}). Alternatively, "sub-critical fluid" refers to a fluid or condensed phase having a sub-critical pressure ratio between about 0.95 and about 1.0, wherein the sub-critical pressure ratio equals the actual pressure divided by the critical temperature (PR_{sub-critical} = P_{actual/}P_{c}).

Unless otherwise expressly specified, all of the numerical ranges, amounts, values and percentages such as those for quantities of materials, times, temperatures, reaction conditions, ratios of amounts, values for molecular weight (whether number average molecular weight Mₙ or weight average molecular weight M_{w}), and others disclosed herein should be understood as modified in all instances by the term "about," if about is not expressly used in combination with said ranges, amounts, values, and percentages herein. Accordingly, unless indicated to the contrary, the numerical parameters set forth in the present disclosure and attached claims are approximations that can vary. At the very least, each numerical parameter should at least be construed in light of the number of reported significant digits and by applying ordinary rounding techniques.

Notwithstanding that the numerical ranges and parameters setting forth the broad scope of the disclosure are approximations, the numerical values set forth in the specific examples are reported as precisely as possible. Any numerical value, however, is inherently somewhat uncertain because of the standard deviation found in its respective testing measurement. Furthermore, when numerical ranges of varying scope are set forth herein, it is contemplated that any combination of these values inclusive of the recited values can be used in accordance with the teachings of the disclosure.

Examples provided herein, including those following "such as" and "e.g.," are considered as illustrative only of various aspects and features of the present disclosure and embodiments thereof, and thus should not alter the scope of any of the referenced terms or phrases. Any suitable equivalents, alternatives, and modifications thereof (including materials, substances, constructions, compositions, formulations, means, methods, conditions, etc.) known and/or available to one skilled in the art can be used or carried out in place of or in combination with those disclosed herein, and are considered to fall within the scope of the present disclosure. Throughout the present disclosure in its entirety, any and all of the one, two, or more features and aspects disclosed herein, explicitly or implicitly, following terms "example", "examples", "such as", "e.g.", and the likes thereof may be practiced in any combinations of two, three, or more thereof (including their equivalents, alternatives, and modifications), whenever and wherever appropriate as understood by one of ordinary skill in the art. Therefore, specific details disclosed herein are not to be interpreted as limiting, but merely as a basis for the claims and as a representative basis for teaching one skilled in the art to variously employ aspects and features of the present disclosure in virtually any appropriate manner as understood by one of ordinary skill in the art.

### Microparticles

Non-limiting microparticles, materials and methods for fabricating microparticles, compositions and formulations containing microparticles, and utilities of such microparticles, compositions, and formulations include those disclosed in U.S. Patent Nos. 5,525,519, 5,554,730, 5,578,709, 5,599,719, 5,981,719, 6,090,925, 6,268,053, and 6,458, 387, U.S. Publication Nos. 20030059474, 20030064033, 20040043077, 20050048127, 20050142201, 20050142205, 20050142206, 20050147687, 20050170005, 20050233945, 20060018971, 20060024240, 20060024379, 20060260777, 20070092452, 20070207210, and 20070281031. Microparticles can have a generally uniform size distribution, such as a monodisperse size distribution, or a polydisperse size distribution, and a generally uniform shape, such as being substantially spherical. One or more characteristics of the microparticles can be adjusted during fabrication by manipulating one or more variables such as, but not limited to, selection of ingredients or combination thereof, concentrations of different ingredients, reaction temperature, reaction time, and/or pH if reaction is taken place in aqueous solution.

Microparticles are suitable for delivering, *in vivo, ex vivo,* and/or *in vitro,* one active agent or a combination of two or more active agents with rapid and/or controlled release profiles, and are useful for a wide variety of therapeutic, pharmaceutical, diagnostic, medical, medicinal, cosmetic, nutritional, biocidic, separational, industrial, commercial, and research applications, such as drug delivery, vaccination, gene therapy and histopathological or *in vivo* tissue or tumor imaging. Microparticles can be formulated for oral, parenteral, mucosal; ophthalmic; intravenous, subcutaneous, subdermal, intradermal, intra-articular, intramuscular, pulmonary (including oral and nasal inhalations), and/or topical administrations to a subject. Intravenous administration includes catheterization and angioplasty.

The microparticles typically contain one or more macromolecules. The one or more macromolecules (typically, one or more bioactive macromolecules and/or one or more carrier macromolecules) may comprise at least 20%, 30%, 40%, 50%, 60%, 70%, 80%, 90%, 95%, or 98%, and up to 100%, or less than 100%, by weight and/or volume of the microparticle, or be present in a range between any two of such values. It will be understood by those skilled in the art that the macromolecule can be a portion (e.g., fragment, segment, subunit) of another larger macromolecule. It will be further understood that macromolecules include affinity molecules, which can be, for example, the receptor or ligand portions of a receptor-ligand interaction. Non-limiting examples of ligands include viruses, bacteria, polysaccharides, or toxins that act as antigens to generate immune responses when administered to an animal and cause the production of antibodies.

One or more ingredients other than the macromolecules described above and the active agents described below including but not limited to polymers, complexing agents, stabilizing agents, excipients, ions, moisture, residual solvents, impurities, by-products, may be present in the microparticle at a quantity of 50% or less, 30% or less, 20% or less, 10% or less, 5% or less, or 2% or less, or greater than 0%, by weight and/or volume of the microparticle, or in a range between any two of such values. Additionally, any ingredients present in the reaction/incubation medium (e.g., such as non-volatile materials) during the formation of the microparticles can be substantially removed from and thus absent in the resulting microparticles. Immediately or at a later stage following their formation (which may or may not be in-situ), the microparticles may be dispersed (e.g., as colloids or suspensions) in a continuous solid phase (e.g., a frozen solid comprising the dispersion) or in a non-solid phase (e.g., a flowable medium, such as the reaction/incubation medium in which the microparticles are formed, or a washing medium).

The microparticles may have a density substantially the same as or different from (such as greater than or less than) that of the continuous phase (measured at the same temperature, such as ambient temperature). Densities of the microparticles, and the continuous phase equal their respective weight divided by their respective volume. The microparticles may have a density less than, equal to, or greater than values such as 0.8 g/cm³, 0.95 g/cm³, 1.0 g/cm³, 1.05 g/cm³, 1.1 g/cm³, 1.3 g/cm³, 1.35 g/cm³, 1.5 g/cm³, and 1.9 g/cm³, or in a range between any two of such values, such as between 1.0 g/cm³ and 1.5 g/cm³ or between 1.2 g/cm³ and 1.5 g/cm³. Density of the microparticles may be measured by helium pycnometry at ambient temperature, by density-gradient techniques (e.g., using centrifugation or ultracentrifugation) using suitable gradient medium (e.g., salts of alkali metals such as NaCl, NaBr, NaI, KBr, CsF, CsCl, CsBr, cesium sulfate, cesium acetate, cesium trifluoroacetate, RbCl, and potassium tartrate; neutral, water-soluble molecules such as sucrose with optional addition of glucose, glycerol, or mineral oil; hydrophilic macromolecules such as dextran, sucrose-epichlorohydrin copolymer, and bovine serum albumin; other synthetic molecules such as sodium or methyl glucamine salts of triiodobenzoic acid and of metrizoic acid, and metrizamide), and other known methods. Standard methods involving density-gradient techniques include ASTM D1505-03, ASTM D1505-98, and ISO 1183-2.

### Active agents

One or more active agents are typically covalently and/or non-covalently associated with, and/or entrapped by, at least a portion (e.g., the center or core, one or more specifically or randomly distributed compartments, inner and/or outer surfaces) of the microparticle. For example, the one or more active agents may be covalently and/or non-covalently associated with, and/or entrapped by, at least a portion or substantially all of one or more macromolecules (e.g., bioactive macromolecules and/or carrier macromolecules) and/or one or more other ingredients (e.g., with one or more polymers, as complexes or conjugates thereof).

The active agent may be a pharmaceutical agent. Depending on its effect and/or application, the pharmaceutical agent includes, without limitation, adjuvants, adrenergic agents, adrenergic blocking agents, adrenocorticoids, adrenolytics, adrenomimetics, alkaloids, alkylating agents, allosteric inhibitors, anabolic steroids, analeptics, analgesics, anesthetics, anorexiants, antacids, anti-allergic agents, antiangiogenesis agents, anti-arrhythmic agents, anti-bacterial agents, antibiotics, antibodies, anticancer agents such as paclitaxel and derivative compounds, anticholinergic agents, anticholinesterases, anticoagulants, anticonvulsants, antidementia agents, antidepressants, antidiabetic agents, antidiarrheals, antidotes, antiepileptics, antifolics, antifungals, antigens, antihelmintics, antihistamines, antihyperlipidemics, antihypertensive agents, anti-infective agents, anti-inflammatory agents, antimalarials, antimetabolites, antimuscarinic agents, antimycobacterial agents, antineoplastic agents, antiosteoporosis agents, antipathogen agents, antiprotozoal agents, adhesion molecules, antipyretics, antirheumatic agents, antiseptics, antithyroid agents, antiulcer agents, antiviral agents, anxiolytic sedatives, astringents, beta-adrenoceptor blocking agents, biocides, blood clotting factors, calcitonin, cardiotonics, chemotherapeutics, cholesterol lowering agents, cofactors, corticosteroids, cough suppressants, cytokines, diuretics, dopaminergics, estrogen receptor modulators, enzymes and cofactors thereof, enzyme inhibitors, growth differentiation factors, growth factors, hematological agents, hematopoietics, hemoglobin modifiers, hemostatics, hormones and hormone analogs, hypnotics, hypotensive diuretics, immunological agents, immunostimulants, immunosuppressants, inhibitors, ligands, lipid regulating agents, lymphokines, muscarinics, muscle relaxants, neural blocking agents, neurotropic agents, parasympathomimetics, parathyroid hormone, promoters, prostaglandins, psychotherapeutic agents, psychotropic agents, radio-pharmaceuticals, receptors, sedatives, sex hormones, sterilants, stimulants, thrombopoietics, trophic factors, sympathomimetics, thyroid agents, vaccines, vasodilators, vitamins, xanthines, as well as conjugates, complexes, precursors, and metabolites thereof. The active agent may be used individually or in combinations of two or more thereof. In one example, the active agent is a prophylactic and/or therapeutic agent that includes, but is not limited to, peptides, carbohydrates, nucleic acids, other compounds, precursors and derivatives thereof, and combinations of two or more thereof. In one aspect, the active agent is a pharmaceutical agent that is conventionally referred to as a small molecule.

The active agent may be a bioactive active agent, for example, a bioactive macromolecule, such as a protein (including the proteinaceous compounds described above), a polypeptide, a carbohydrate, a polynucleotide, a vector (e.g., a virus or viral particle), or a nucleic acid, or a combination of two or more thereof. The macromolecule can be natural or synthetic. Exemplary proteins include monoclonal antibodies and polyclonal antibodies. The protein can also be any known therapeutic proteins isolated from natural sources or produced by synthetic or recombinant methods. Examples of therapeutic proteins include, but are not limited to, proteins of the blood clotting cascade (e.g., Factor VII, Factor VIII, Factor IX, et al.), subtilisin, ovalbumin, alpha-1-antitrypsin (AAT), DNase, superoxide dismutase (SOD), lysozyme, ribonuclease, hyaluronidase, collagenase, growth hormone, erythropoietin, insulin-like growth factors or their analogs, interferons, glatiramer, granulocyte-macrophage colony-stimulating factor, granulocyte colony-stimulating factor, antibodies, PEGylated proteins, glycosylated or hyperglycosylated proteins, desmopressin, LHRH agonists such as: leuprolide, goserelin, nafarelin, buserelin; LHRH antagonists, vasopressin, cyclosporine, calcitonin, parathyroid hormone, parathyroid hormone peptides and insulin.

The active agent may be a cosmetic agent. Non-limiting examples of cosmetic agents include emollients, humectants, free radical inhibitors, anti-inflammatory agents, vitamins, depigmenting agents, anti-acne agents, antiseborrhoeics, keratolytics, slimming agents, skin coloring agents, and sunscreen agents. Non-limiting compounds useful as cosmetic agents include linoleic acid, retinol, retinoic acid, ascorbic acid alkyl esters, polyunsaturated fatty acids, nicotinic esters, tocopherol nicotinate, unsaponifiables of rice, soybean or shea, ceramides, hydroxy acids such as glycolic acid, selenium derivatives, antioxidants, beta-carotene, gamma-orizanol, and stearyl glycerate. The cosmetic agents may be commercially available and/or prepared by known techniques. As above, the various active agents may be used individually or in combinations of two or more thereof.

The active agent may be a nutritional supplement. Non-limiting examples of nutritional supplements include proteins, carbohydrates, water-soluble vitamins (e.g., vitamin C, B-complex vitamins, and the like), fat-soluble vitamins (e.g., vitamins A, D, E, K, and the like), and herbal extracts. The nutritional supplements may be commercially available and/or prepared by known techniques. As above, the various active agents may be used individually or in combinations of two or more thereof.

The active agent may be a compound having a molecular weight of 2kD or less. Non-limiting examples of such compounds include steroids, beta-agonists, anti-microbial agents, antifungal agents, taxanes (antimitotic and antimicrotubule agents), amino acids, aliphatic compounds, aromatic compounds, and urea compounds. Active agents conventionally known as small molecules (or small organic molecules) are representative active agents having a molecular weight of 2kD or less.

The active agent may also be a diagnostic agent. Non-limiting diagnostic agents include x-ray imaging agents and contrast media. Non-limiting examples of x-ray imaging agents include ethyl 3,5-diacetamido-2,4,6-triiodobenzoate (WIN-8883, ethyl ester of diatrazoic acid); 6-ethoxy-6-oxohexyl-3,5-bis(acetamido)-2,4,6-triiodobenzoate (WIN 67722); ethyl-2-(3,5-bis(acetamido)-2,4,6-triiodobenzoyloxy)-butyrate (WIN 16318); ethyl diatrizoxyacetate (WIN 12901); ethyl 2-(3,5-bis(acetamido)-2,4,6-triiodobenzoyloxy)propionate (WIN 16923); N-ethyl 2-(3,5-bis(acetamido)-2,4,6-triiodobenzoyloxy-acetamide (WIN 65312); isopropyl 2-(3,5-bis(acetamido)-2,4,6-triiodobenzoyloxy)acetamide (WIN 12855); diethyl 2-(3,5-bis(acetamido)-2,4,6-triiodobenzoyloxymalonate (WIN 67721); ethyl 2-(3,5-bis(acetamido)-2,4,6-triiodobenzoyloxy)phenyl-acetate (WIN 67585); propanedioic acid, [[3,5-bis(acetylamino)-2,4,5-triodobenzoyl]oxy]bis(1-methyl)ester (WIN 68165); and benzoic acid, 3,5-bis(acetylamino)-2,4,6-triodo-4-(ethyl-3-ethoxy-2-butenoate)ester (WIN 68209). Preferred contrast agents desirably disintegrate relatively rapidly under physiological conditions, thus minimizing any particle associated inflammatory response. Disintegration may result from enzymatic hydrolysis, solubilization of carboxylic acids at physiological pH, or other mechanisms. Thus, poorly soluble iodinated carboxylic acids such as iodipamide, diatrizoic acid, and metrizoic acid, along with hydrolytically labile iodinated species such as WIN 67721, WIN 12901, WIN 68165, and WIN 68209 or others may be preferred.

In one specific embodiment, the active agent may be a therapeutic agent for prevention and/or treatment of pulmonary disorders. Non-limiting examples of such agents include steroids, beta-agonists, anti-fungal agents, anti-microbial compounds, bronchial dialators, anti-asthmatic agents, non-steroidal anti-inflammatory agents (NSAIDS), AAT, and agents to treat cystic fibrosis. Non-limiting examples of steroids for prevention and/or treatment of pulmonary disorders include but are not limited to beclomethasone (such as beclomethasone dipropionate), fluticasone (such as fluticasone propionate), budesonide, estradiol, fludrocortisone, flucinonide, triamcinolone (such as triamcinolone acetonide), flunisolide, and salts thereof. Non-limiting examples of beta-agonists for prevention and/or treatment of pulmonary disorders include salmeterol xinafoate, formoterol fumarate, levo-albuterol, bambuterol, tulobuterol, and salts thereof. Non-limiting examples of anti-fungal agents for prevention and/or treatment of pulmonary disorders include itraconazole, fluconazole, amphotericin B, and salts thereof.

The active agents may be used in a combination of two or more thereof. Non-limiting exemplary combinations include a steroid and a beta-agonist, e.g., fluticasone propionate and salmeterol, budesonide and formoterol, etc. Many other viable therapeutically active agent combinations are well known to those of ordinary skill in the art.

### Continuous Phase

The continuous phase of the multi-phasic dispersion may be non-solid, for example, a liquid phase containing one solvent or a homogeneous mixture of two or more solvents (wherein at least a first solvent is soluble in or miscible with at least a second solvent), or a multiple phase system including at least two immiscible solvents. Non-limiting examples of the solvents include aqueous fluids (e.g., water H₂O, D₂O, aqueous buffers, and other aqueous solutions), non-aqueous fluids (e.g., organic solvents, organic buffers), and combinations of two or more of the foregoing. In one aspect, the non-solid continuous phase may be substantially aqueous, for example, containing more than 10% by volume, such as 25% or more, 50% or more, or 75%, or more water. The continuous phase is either partially or completely aqueous or aqueous-miscible.

The continuous phase or the liquid phase(s) thereof may have a density at ambient temperature that is less than or equal to values such as 1.10 g/cm³, 1.05 g/cm³, 1.0 g/cm³, 0.95 g/cm³, 0.9 g/cm³, 0.8 g/cm³, 0.7 g/cm³, 0.6 g/cm³, or in a range between any two of such values. When measured at the same ambient temperature, such as 20°C or 25°C, the continuous phase or liquid phase(s) typically have a density similar or equal to, or less than that of the dispersed phase or the microparticles therein. Most typically, the continuous phase or the liquid phase(s) have a density less than that of the microparticles.

The continuous phase may further contain one or more ingredients dissolved and/or dispersed therein including but not limited to ionic and/or non-ionic polymers, salts, ions, excess reagents, excipients (e.g., sugars, polyols, surfactants), and/or manufacturing-related compounds. Non-limiting examples of salts include ammonium acetate, ammonium bicarbonate, and other buffer salts known to one of ordinary skill in the art. Non-limiting examples of sugars include trehalose, sucrose, lactose, and other carbohydrates known to one of ordinary skill in the art. Non-limiting examples of polyols include mannitol and other sugar alcohols known to one of ordinary skill in the art. The one or more fluids and/or solutes of the continuous phase may be, independently, partially or fully aqueous-miscible, aqueous-immiscible, water-soluble, and/or water-insoluble.

The continuous phase typically contains at least one non-volatile material solubilized therein. The continuous phase, for example, the solvent thereof and/or the non-volatile material therein may be soluble in and/or miscible with the super critical fluid or sub-critical fluid, for example, having a solubility therein at ambient temperature of 10% by weight or greater, such as equal to or greater than values such as 15%, 20%, 25%, 30%, 40%, 50%, 60%, 70%, 80%, 90%, 100%, or in a range between any two of such values. Additionally, the continuous phase, the solvent thereof and/or the non-volatile material therein may be entirely miscible with the super critical fluid or sub-critical fluid. Greater solubility values for the continuous phase and/or components thereof in the super critical fluid or sub-critical fluid are generally preferred as such greater solubilities facilitate removal of the continuous phase from the dispersions in accordance with the disclosed methods.

### Non-volatile material

As previously indicated, at least the continuous phase typically contains at least one non-volatile material such as a non-ionic polymer solubilized therein, the one or more non-volatile materials being different in their chemical structures and/or compositions from that of the one or more macromolecules that form the microparticles. It should be noted that the non-volatile material may be present on/in the dispersed phase, too, for example, the non-volatile material may be trapped within pores of the microparticles and/or otherwise associated with the microparticles.

Generally, the non-volatile materials have a boiling point and/or a flash point greater than about 100°C, greater than about 150°C, and/or greater than about 200°C. The non-volatile materials can be natural, synthetic, semi-synthetic, or recombinant. The one or more non-volatile materials are non-ionic polymers which can be independently aqueous-soluble (e.g., water-soluble), and/or aqueous-miscible (e.g., water-miscible). The non-ionic polymers can also be hydrophilic and/or amphiphilic. The one or more non-volatile materials can beneficially independently or collectively reduce the solubility of one or more macromolecules in the continuous phase (and thus of the microparticles formed therefrom), or in the one or more solvents therein. The one or more non-volatile materials, when present in the continuous phase, typically do not covalently and/or ionically interact with, or denature, the one or more macromolecules in the microparticles. Additionally, the one or more non-volatile materials, when present in the continuous phase, typically do not complex, conjugate, aggregate, and/or agglomerate with each other, or otherwise come together, such as via covalent, ionic, and/or other interactions. Further, the one or more non-volatile materials in the continuous phase typically do not undergo gelation (e.g., form a hydrogel), either by themselves or with other ingredients present in the continuous phase. The one or more non-volatile materials independently generally have molecular weights greater than or equal to values such as 200 daltons, 300 daltons, 400 daltons, 600 daltons, 800 daltons, 1,000 daltons, 1,500 daltons, 2,000 daltons, 2,500 daltons, 3,000 daltons, 3,500 daltons, 4,000 daltons, 5,000 daltons, 8,000 daltons, and 10,000 daltons, or up to about 3,000 kilodaltons (kd), or in a range between any two of such values, for example, between 1000 daltons and 1500 daltons, between 1000 daltons and 2,000 daltons, between 1000 daltons and 2,500 daltons, between 1000 daltons and 3,000 daltons, between 1000 daltons and 3,500 daltons, between 1000 daltons and 4,000 daltons, between 1000 daltons and 5,000 daltons, between 1000 daltons and 8,000 daltons, between 1000 daltons and 10,000 daltons, between 1,500 daltons and 2,000 daltons, between 1,500 daltons and 2,500 daltons, between 1,500 daltons and 3,000 daltons, between 1,500 daltons and 3,500 daltons, between 1,500 daltons and 4,000 daltons, between 1,500 daltons and 5,000 daltons, between 1,500 daltons and 8,000 daltons, between 1,500 daltons and 10,000 daltons, between 2,000 daltons and 2,500 daltons, between 2,000 daltons and 3,000 daltons, between 2,000 daltons and 3,500 daltons, between 2,000 daltons and 4,000 daltons, between 2,000 daltons and 5,000 daltons, between 2,000 daltons and 8,000 daltons, between 2,000 daltons and 10,000 daltons.

Non-limiting examples of non-volatile materials for the continuous phase include the non-ionic water-soluble and/or water-miscible polymers disclosed in U.S. Patent Nos. 5,525,519, 5,554,730, 5,578,709, 5,599,719, 5,981,719, 6,090,925, 6,268,053, and 6,458, 387, U.S. Publication Nos. 20030059474, 20030064033, 20040043077, 20050048127, 20050142201, 20050142205, 20050142206, 20050147687, 20050170005, 20050233945, 20060018971, 20060024240,20060024379,20060260777,20070092452,20070207210,and 20070281031. Non-limiting examples of suitable non-volatile materials may be linear, branched, or cyclic, and include non-ionic polyethers, non-ionic copolyethers, non-ionic polyesters, non-ionic copolyesters, non-ionic polyether-polyester copolymers, non-ionic vinyl polymers, non-ionic pyrrolidone-containing polymers, non-ionic polymeric carbohydrates, derivatives and salts thereof, and combinations of two or more thereof. Non-limiting examples of non-ionic polyethers and non-ionic copolyethers (including copolymers and terpolymers) include but are not limited to hydroxy-terminated polyethers (e.g., polyether alcohols, polyether polyols, ethylene oxide end-capped polyethers other than polyethylene glycols) and alkyl (e.g., methyl, ethyl, propyl, butyl, etc.) end-capped derivatives thereof, such as polyalkylene glycols (e.g., polyoxy-1,2-alkylene glycols like polyethylene glycols and polypropylene glycols, as well as polytrimethylene ether glycols and polytetramethylene ether glycols), hydroxy-terminated copolyethers (e.g., copolyether alcohols, copolyether polyols, ethylene oxide end-capped copolyethers) and alkyl (e.g., methyl, ethyl, propyl, butyl, etc.) end-capped derivatives thereof, such as block copolyethers of two or more different 1,2-alkylene oxides (e.g., polyoxyethylene-polyoxypropylene copolymers like poloxamers) and copolyethers of one or more 1,2-alkylene oxides and one or more of tetrahydrofuran, tetrahydropyran, and 1,3-propanediol (e.g., (polyethylene glycol)-(polytrimethylene ether glycol) copolymers, (polyethylene glycol)-(polytetramethylene ether glycol) copolymers). Non-limiting examples of non-ionic polyesters and non-ionic copolyesters (including copolymers and terpolymers) include hydroxy-terminated polyesters (e.g., polyester polyols, copolyester polyols, ethylene oxide end-capped or polyoxyethylene-terminated polyesters, and certain silicone polyesters, such as the likes ofpolyoxyethylene glycerin dicarboxylic acid esters, polyoxyethylenesorbitol dicarboxylic acid esters, polyoxyethylene glycol dicarboxylic acid esters, and polyoxyethylenealkyl esters. Non-limiting examples of non-ionic polyether-polyester copolymers (including terpolymers) include but are not limited to block copolymers of one or more lactones and/or dicarboxylic acids and one or more 1,2-alkylene oxides), esterification derivatives of the non-ionic polyethers and non-ionic copolyethers disclosed herein, and etherification derivatives of the non-ionic polyesters and non-ionic copolyesters disclosed herein, such as (polyethylene glycol)-polycaprolactone block copolymers. Non-limiting examples of non-ionic vinyl polymers (including copolymers and terpolymers) and pyrrolidone-containing non-ionic polymers (including copolymers and terpolymers) include but are not limited to polyvinyl alcohols, homopolymers and copolymers (including terpolymers) of hydroxyalkyl (alk)acrylates (e.g., hydroxyethyl acrylate, hydroxypropyl acrylate, hydroxyethyl methacrylate, hydroxypropyl methacrylate), oligo-oxyalkylene (alk)acrylates (e.g., oligo-oxyethylene acrylates, oligo-oxyethylene methacrylates), and/or alkyl end-capped oligo-oxyalkylene (alk)acrylates (e.g., methyl-capped), polyvinylpyrrolines, and (alkenyl pyrrolidone)-containing homopolymers and copolymers.

Non-limiting examples of non-ionic polymeric (including oligomeric) carbohydrates (having a molecular weight of 200 daltons to 5,000,000 daltons, such as 500 daltons, 1,000 daltons, 3,000 daltons, 5,000 daltons, 10,000 daltons, 30,000 daltons, 50,000 daltons, 100,000 daltons, 300,000 daltons, 500,000 daltons, 1,000,000 daltons, or 3,000,000 daltons, or in a range between any two of such values) and derivatives thereof include but are not limited to starch, amylopectin (branched polysaccharides), amylose (linear polysaccharides), cellulose, guar gum, guar polysaccharides, xanthan gum, dextrins (e.g, cyclodextrins, maltodextrins), dextrans, polydextroses, gellan gum, pullulan, cellodextrins, beta-glucans, and derivatives thereof, for example, non-ionic esters formed by esterification, including but not limited to benzoates and alkanoates such as acetates, propionates, butyrates, and hexanoates; or non-ionic ethers formed by etherification such as non-ionic starch ethers, non-ionic amylopectin ethers, non-ionic amylose ethers, non-ionic cellulose ethers, non-ionic guar ethers, non-ionic starch esters, non-ionic amylopectin esters, non-ionic amylose esters, non-ionic cellulose esters, non-ionic starch ether esters, non-ionic starch ester ethers, non-ionic cellulose ether esters, and non-ionic cellulose ester ethers. Non-limiting examples of non-ionic starch ethers include alkylstarches such as methylstarches, ethylstarches, propylstarches, and butylstarches; hydroxyalkyl starches such as hydroxyethyl starches (e.g., tetrastarch, pentastarch, hetastarch), hydroxypropyl starches, hydroxybutyl starches, and hydroxypentyl starches; as well as alkylhydroxylalkyl starches such as methylhydroxyethyl starches, methylhydroxypropyl starches, and ethylhydroxypropyl starches. Non-limiting examples of non-ionic amylopectin ethers and non-ionic amylose ethers include hydroxyethyl amylopectins, hydroxypropyl amylopectins, hydroxyethyl amyloses, and hydroxypropyl amyloses. Non-limiting examples of non-ionic cellulose ethers include alkylcelluloses such as methylcelluloses, ethylcelluloses, propylcelluloses, isopropylcelluloses, and butylcelluloses; hydroxyalkyl celluloses such as hydroxyethyl celluloses, hydroxypropyl celluloses, hydroxyisopropyl celluloses, hydroxybutyl celluloses, and hydroxypentyl celluloses; as well as alkylhydroxylalkyl celluloses such as methylhydroxyethyl celluloses, methylhydroxypropyl celluloses, methylhydroxybutyl celluloses, ethylhydroxyethyl celluloses, ethylhydroxypropyl celluloses, propylhydroxyethyl celluloses, propylhydroxypropyl celluloses, isopropylhydroxypropyl celluloses, butylhydroxypropyl celluloses, pentylhydroxypropyl celluloses, and hexylhydroxypropyl celluloses. Non-limiting examples of non-ionic guar ethers include alkylguar polysaccharides such as methylguar polysaccharides, ethylguar polysaccharides, propylguar polysaccharides, and butylguar polysaccharides; hydroxyalkylguar polysaccharides such as hydroxyethylguar polysaccharides, and hydroxypropylguar polysaccharides; as well as alkylhydroxylalkylguar polysaccharides such as methylhydroxyethylguar polysaccharides, methylhydroxypropylguar polysaccharides, ethylhydroxypropylguar polysaccharides. Other non-ionic polymeric carbohydrates include methyl cellulose, hydroxyethyl cellulose, hydroxyethyl methylcellulose, hydroxypropyl methylcellulose, ethylhydroxyethyl cellulose, methylethylhydroxyethyl cellulose, butylglycidyletherhydroxyethyl cellulose, laurylglycidyletherhydroxyethyl cellulose, hydroxymethylhydroxyethyl cellulose, butylglycidylether modified hydroxyethyl cellulose, methylhydroxyethylcellulose, methylhydroxypropyl cellulose, starch esters (e.g. alkyl succinic anhydride modified starchstarch acetates and starch alkenylsuccinates), cellulose esters (cellulose monobutyrates and monopropionates), cellulose ether esters (hydroxyalkyl cellulose-2-hydroxycarboxylic acid esters), poly(3-hydroxyoxetane)s. Non-limiting examples of non-ionic polymeric carbohydrate esters include those having a degree of substitution ranging from 0.5 to 1.0, such as from 0.7 to 0.9, and are water-soluble. Ionic salts of the foregoing non-ionic materials, if capable of being made, may also be used. For example, salts of polysaccharides such as dextran sulfate, dextrin sulfate, and sodium alginate, can also be used.

### Dispersed Phase

The dispersed phase of the multi-phasic dispersion may comprise solid microparticles. Typically, it is preferred that the microparticles are substantially insoluble in and/or substantially immiscible with the super critical fluid or sub-critical fluid, for example, having a solubility therein at ambient temperature of less than 10% by weight, such as 5 wt.% or less, 3 wt.% or less, 1 wt.% or less, 0.5 wt.% or less, 0.1 wt.% or less, 0.05 wt.% or less, 0.01 wt.% or less, or in a range between any two of such values.

The dispersed phase may further include other materials in association with the solid microparticles, for example, a non-volatile material, salt, or excipient added during microparticle formation. Generally, such materials are not desired in the isolated microparticles and are therefore desirably removed from the dispersion. Accordingly, it is desirable for such materials to have greater solubilities in the super critical fluid or sub-critical fluid as described above in the continuous phase section.

### Apparatus for Separating the Microparticles

As depicted in Figure 1, a multi-phasic dispersion including dispersed and continuous phases can be positioned within a chamber or vessel 10 capable of being pressurized so it can be contacted with a gas and/or a fluid to enable and/or facilitate the separation of the continuous phase from the dispersion (or the microparticles therein) to provide microparticles that are substantially free of the non-volatile material and/or the solvent. Typically, the dispersion is positioned within an extraction basket 20 (or similar structure capable of retaining the microparticles while allowing any liquids containing the non-volatile material and/or other undesirable components such as solvents, excipients, salts, and other solutes to be separated therefrom) to facilitate contact with the gas and/or fluid. The extraction basket 20 is typically formed from an inert material such as stainless steel. A representative extraction basket structure 20 includes a hollow sleeve cylinder with solid poreless side walls and two identical stainless steel disk sieves (for example, having a pore size between 0.2 microns and 0.5 microns, or larger such as 1, 2, 3, 4, or 5 microns) coupled to both ends of the extraction basket (e.g., mounted with stainless steel caps or plates or clamps and bolts) to permit entry and egress of the gas and/or fluid. Alternatively, the extraction basket 20 can be composed of two cylindrical cups having open ends capable of mating together (for example, by threads and reciprocal grooves) to form an enclosed cylinder with suitable disk sieves 30 mounted on the bottom of the cups. The size and volume of the basket 20 is only practicably limited by the size of the vessel 10. However, the size of the pores of the disk sieves 30 should be sufficiently small to adequately retain the microparticles of the dispersion during the contacting step(s).

### Contacting the Continuous Phase with a Pressurizing Gas

The multi-phasic dispersion is typically initially contacted with a gas at an elevated pressure such that at least a portion of any liquid associated with the dispersed phase can be forced through the sieve 30, thereby separating at least a portion of the non-volatile material and solvent from the dispersion so as to concentrate the microparticles, which are retained by the sieve 30. The multi-phasic dispersion may be concentrated into a relatively more viscous dispersion prior to being positioned within the chamber by performing a concentration step such as diafiltration or centrifugation on the initial dispersion, but it is not necessary to carry out such a concentration step.

The pressurizing gas is introduced into the chamber via gas inlet valve 40. Suitable gases include carbon dioxide, nitrogen, compressed air, and mixtures thereof. The various gases can be mixed off-line and introduced into the system as a mixture, or can be mixed on line via suitable apparatus (not shown).

The pressure of the pressurizing gas(es) within the vessel 10 is typically greater than or equal to 10 bar but also less than the supercritical pressure of the gas. Because the supercritical pressure of a gas varies with composition, the high end of suitable pressures for the contacting step can vary. The supercritical pressures of many gases and mixtures thereof are well known or can be reasonably determined or estimated using known methods [see, for example, Kroschwitz, J., exec. ed., Kirk-Othmer Encyclopedia of Chemical Technology, pp. 452-477, volume 23, 4th ed., John Wiley and Sons, New York (1997)]. Typically, the chamber 10 is pressurized to a pressure greater than or equal to 10 bar, 15 bar, 20 bar, 25 bar, 30 bar, 35 bar, 40 bar, 45 bar, 50 bar, 55 bar, and/or 60 bar, or any range between these disclosed values, for example, between 10 bar and 60 bar, between 10 bar and 55 bar, between 10 bar and 50 bar, between 10 bar and 45 bar, between 10 bar and 40 bar, between 10 bar and 30 bar, between 10 bar and 25 bar, between 10 bar and 20 bar, and/or between 10 bar and 15 bar, or between 15 bar and 60 bar, between 15 bar and 55 bar, etc. Pressure gauge 50 is used to monitor the pressure of the chamber 10. The gas pressure is maintained for at least 2 seconds, 5 seconds, 10 seconds, 15 seconds, 20 seconds, 30 seconds, 45 seconds, one minute, two minutes, 5 minutes, 10 minutes and/or up to one hour, two hours, or any range between these disclosed values, for example, between 2 seconds and 2 hours and/or between 5 seconds and two minutes.

The selected pressurizing gas(es) can be heated (or cooled) with a suitable heat exchanger (not shown) as appropriate and flowed into chamber 10 at a temperature less than its supercritical temperature. Supercritical temperatures are well known or can be reasonably estimated or determined using known techniques [see, for example, Kroschwitz, J., exec. ed., Kirk-Othmer Encyclopedia of Chemical Technology, pp. 452-477, volume 23, 4th ed., John Wiley and Sons, New York (1997)]. A temperature indicator and controller 60 can be used to monitor the temperature within the chamber 10. Typically, the pressurizing gas temperature is greater than or equal to 0°C, 5°C, 10°C, 15°C, 20°C, 25°C, 30°C, or any range between these disclosed values, for example, between 0°C and 30°C, between 0°C and 25°C, between 0°C and 20°C, between 0°C and 15°C, between 0°C and 10°C, between 0°C and 5°C, between 5°C and 30°C, between 5°C and 25°C, between 5°C and 20°C, between 5°C and 15°C, between 5°C and 10°C, etc.

When the multi-phasic dispersion is contacted with the pressurizing gas, at least a portion of any liquid of the dispersion can be forced through the sieve 30 by the positive gas pressure, thereby separating at least a portion of the non-volatile material and/pr solvent from the dispersion, which is retained by the sieve 30. Accordingly, after the contacting is completed, the gas inlet valve 40 can be shut off, and a valve 70, for example, a ball valve, at the bottom of the chamber 10 can be opened to at least partially depressurize the system and drain any liquid collected therein. Additional contacting steps wherein the gas is pressurized to a pressure, for example, greater than or equal to 10 bar but below the supercritical pressure of the gas, can be carried out until no (including substantially no) additional liquid flows from valve 70 upon opening same. The pressurizing gas(es) used in the additional contacting steps can be the same or different from the pressurizing gas(es) used in the first contacting step.

### Contacting the Dispersion with an Extracting Fluid

When substantially no additional liquid flows from valve 70, the chamber can optionally be repressurized with a second gas, which may be the same or different from the gas used in the prior contacting steps (using pressurizing gas), to a pressure greater than or equal to its supercritical pressure so as to provide an extracting fluid in the form of a supercritical fluid or a sub-critical fluid. For example, the chamber 10 is typically pressurized to a pressure greater than or equal to 70 bar, 80 bar, 90 bar, 100 bar, 150 bar, 200 bar, 225 bar, 250 bar, 275 bar, and/or 300 bar, or any range between these disclosed values, for example, between 70 bar and 300 bar, between 70 bar and 275 bar, between 70 bar and 250 bar, between 70 bar and 225 bar, between 70 bar and 200 bar, between 70 bar and 150 bar, etc.. The gas pressure is maintained for at least 2 seconds, at least 5 seconds, at least 10 seconds, at least 15 seconds, at least 20 seconds, at least 30 seconds, at least 45 seconds, at least one minute, at least two minutes, at least 5 minutes, and/or up to 10 minutes, up to one hour, up to two hours, or any range between these disclosed values, for example, between 2 seconds and 2 hours, between 2 seconds and 1 hour, between 2 seconds and 10 minutes, between 2 seconds and two minutes, etc.

The employed gas can also be heated (as is typically necessary for it to take the form of a supercritical or a sub-critical fluid). For example, the gas may be heated to a temperature greater than its supercritical temperature. Surprisingly, contacting microparticles comprising active agents (for example, bioactive macromolecules) with a supercritical or a sub-critical fluid does not lead to appreciable degradation of the active agent. Such degradation is prevented by controlling the temperature of the system; the gas should not be heated to a temperature at which the formed fluid (supercritical or sub-critical) will degrade and/or denature the active agent in the microsphere. Such degradation temperatures will vary depending on the selected active agent(s) in the microspheres and the gas(es) used to provide the supercritical or sub-critical fluid, but can be easily ascertained by one of ordinary skill in the art with routine experimentation. Typically, the gas temperature is greater than or equal to 20°C, 22.5°C, 25°C, 27.5°C, 30°C, 32.5°C, 35°C, 37.5°C, 40°C, or any range between these disclosed values, for example, between 20°C and 37.5°C, between 20°C and 25°C, etc. Of course, the supercritical temperature of a gas varies with its composition so other values may also be appropriate.

As with the pressurizing gas described above, the gas(es) forming the supercritical or sub-critical fluid is introduced into the chamber via gas inlet valve 40. The gas may be a single gas or a mixture of two or more gases. Representative gases include carbon dioxide, isopropanol, methanol, ethanol, water, toluene, ethylene, xenon, ethane, dimethyl ether, nitrous oxide, propane, ammonia, butane, pentane, and mixtures thereof. A preferred gas is dimethylether because of its dipole moment. Dimethylether is typically provided as a sub-critical fluid for the reasons described above relating to the degradation of active agent within the microspheres because of its relatively high super critical temperature (127°C). A preferred mixture comprises carbon dioxide and a second co-solvent component such as ethanol or isopropanol in a volume or weight ratio between 1:99 and 99:1, between 5:95 and 95:5, for example, 70:30 carbon dioxide/ethanol. The various gases can be mixed off-line and introduced into the system as a mixture, or can be mixed on line via suitable apparatus (as described above).

When this step is carried out, a supercritical or sub-critical fluid is provided within the chamber to contact a remaining portion of the multi-phasic dispersion, thereby separating substantially all of the remaining or residual non-volatile material and/or solvent and other components from the multi-phasic dispersion, and providing microparticles that are substantially free of non-volatile material, salts, excipients, and/or other solutes. For example, the obtained microparticles can contain less than 5 wt.% non-volatile material, less than 4 wt.% non-volatile material, less than 3 wt.% non-volatile material, less than 2 wt.% non-volatile material, less than 1 wt.% non-volatile material, less than 0.5 wt.% non-volatile material, less than 0.25 wt.% non-volatile material, or any range between these disclosed values, for example, between 0.25 wt.% and 5 wt.% non-volatile material, between 0.25 wt.% and 4 wt.% non-volatile material, between 0.25 wt.% and 3 wt.% non-volatile material, between 0.25 wt.% and 2 wt.% non-volatile material, between 0.25 wt.% and 1 wt.% non-volatile material, between 0.25 wt.% and 0.5 wt.% non-volatile material, between 0.5 wt.% and 5 wt.% non-volatile material, between 0.5 wt.% and 4 wt.% non-volatile material, between 0.5 wt.% and 3 wt.% non-volatile material, between 0.5 wt.% and 2 wt.% non-volatile material, between 0.5 wt.% and 1 wt.% non-volatile material, etc. Similarly, the obtained microparticles contain less than 5 wt.% solvent, less than 4 wt.% solvent, less than 3 wt.% solvent, less than 2 wt.% solvent, less than 1 wt.% solvent, less than 0.5 wt.% solvent, less than 0.25 wt.% solvent, or any range between these disclosed values, for example, between 0.25 wt.% and 5 wt.% solvent, between 0.25 wt.% and 4 wt.% solvent, between 0.25 wt.% and 3 wt.% solvent, between 0.25 wt.% and 2 wt.% solvent, between 0.25 wt.% and 1 wt.% solvent, between 0.25 wt.% and 0.5 wt.% solvent, between 0.5 wt.% and 5 wt.% solvent, between 0.5 wt.% and 4 wt.% solvent, between 0.5 wt.% and 3 wt.% solvent, between 0.5 wt.% and 2 wt.% solvent, between 0.5 wt.% and 1 wt.% solvent, etc.

After the contacting step is conducted, a third gas, for example, a humidified gas such as humidified nitrogen, humidified air, or humidified noble gases, can optionally be directed or passed over the obtained microparticles to beneficially exchange any remaining residual solvent therein with water. Specifically, the microparticles are placed in a supporting member (e.g., vials, trays, pans, dishes), which is in turn placed in a chamber (e.g., dry box, lyophilizer) under a stream of the humidified gas. Contacting the microparticles with a humidified gas having a relative humidity of 25% to 100%, such as 30% to 95%, 40% to 90%, or 50% to 80%, facilitates the evaporation of residual solvent from the microparticles. Following the high humidity evaporation process, which may last from hours (e.g., 10, 12, 20 hrs) to days depending on the volume or weight of the microparticle composition, the residual solvent content therein can be further reduced. If the residual solvent is not water miscible, a gas containing little to no humidity can be used to achieve similar benefits.

As discussed above, preferably, the dispersed phase or at least the microparticles therein are insoluble in the supercritical or sub-critical fluid, and the continuous phase or at least one solvent or non-volatile material therein is soluble in or miscible with the supercritical fluid. The continuous phase of the dispersion as a whole, or one or more of the continuous phase components (such as a liquid, like water, and/or a solute, like a polymer) therein, is therefore preferably more soluble in and/or miscible with the supercritical fluid than the microparticles. As such, the one or more supercritical fluids may be suitable as differential solubility systems to separate (e.g., extract, wash, exclude, displace, remove) the continuous phase or at least one or more components therein from the microparticles. In one example, the continuous phase, which can be aqueous, organic, and/or multi-component (e.g., binary, ternary) is partially or fully miscible with the supercritical fluid. In another example, the one or more solutes (such as one or more non-ionic polymers) in the continuous phase have a solubility in the supercritical fluid of 10% by weight or greater, such as less than, equal to, or greater than values such as 15%, 20%, 25%, 30%, 40%, 50%, 60%, 70%, 80%, 90%, 100%, or in a range between any two of such values.

The following examples are provided to illustrate the invention, but is not intended to limit the scope thereof.

### EXAMPLE 1

This example discloses a process to remove at least a portion of a non-volatile material and./or a solvent from a dispersion comprising microparticles to thereby harvest microparticles substantially free of non-volatile material and/or solvent in a single process.

Specifically, a dispersion containing a dispersed phase of IGIV microparticles in a continuous phase comprising a non-volatile material (PEG 300) was loaded into a stainless steel extraction basket, and placed in a supercritical fluid vessel chamber. The SCF chamber was then sealed and pressurized to 40 bar with carbon dioxide. The CO₂ supply was turned off to partially depressurize the SCF chamber (the pressure was dropped to 20-30 bar), and a ball valve at the bottom of the SCF chamber was opened slightly. At this point, a liquid, which generally comprised the non-volatile material, filtered through the extraction basket under the increased gas pressure of the chamber, and was removed from the system through the opened ball valve. The sub-critical gas pressurization and subsequent liquid draining were repeated until no additional liquid drained from the system upon opening the ball valve. The SCF chamber was then sealed, and a SCF/co-solvent extraction process was initiated to remove any residual continuous phase, under the following conditions: 98 wt.% of CO₂ with 2 wt.% ethanol at a flow rate of 50 g/min (up-flow), 250 bar, and 35°C.

In this example, IGIV microspheres, which were substantially free of non-volatile material were obtained.

### EXAMPLE 2

This example also discloses a process to remove at least a portion of a non-volatile material and/or a solvent from a dispersion comprising microparticles to thereby harvest microparticles substantially free of non-volatile material and/or solvent in a single process.

A dispersion containing a dispersed phase of IGIV microparticles in a continuous phase comprising a non-volatile material (PEG 3350) and water was loaded into a stainless steel extraction basket, and placed in a supercritical fluid vessel chamber. The SCF chamber was then sealed and pressurized to 40 bar with carbon dioxide. The CO₂ supply was turned off to partially depressurize the SCF chamber (the pressure was dropped to 20-30 bar), and a ball valve at the bottom of the SCF chamber was opened slightly. At this point, a liquid, which generally comprised the non-volatile material and water, filtered through the extraction basket under the increased gas pressure of the chamber, and was removed from the system through the opened ball valve. The sub-critical gas pressurization and subsequent liquid draining were repeated until no additional liquid drained from the system upon opening the ball valve. The SCF chamber was then sealed, and a SCF/co-solvent extraction process was initiated to remove any residual continuous phase and/or solvent, under the following conditions: 70 wt.% CO₂ with 30 wt.% ethanol at a total flow rate of 100 g/min (up-flow), 250 bar, and 20 °C.

In this example, IGIV microspheres, which were substantially free of solvent and non-volatile material were obtained.

### EXAMPLE 3

This prophetic example discloses a process to remove at least a portion of a non-volatile material and/or a solvent from a dispersion comprising microparticles to thereby harvest microparticles substantially free of non-volatile material and/or solvent in a single process.

A dispersion containing a dispersed phase of insulin microparticles in a continuous phase comprising a non-volatile material (poloxamer 188 or PEG 300 or tetraglycol) and water is loaded into a stainless steel extraction basket, and placed in a supercritical fluid vessel chamber. The SCF chamber is then sealed and pressurized to 30 bar with carbon dioxide. The CO₂ supply is turned off to partially depressurize the SCF chamber (the pressure is dropped to 10-20 bar), and a ball valve at the bottom of the SCF chamber is opened slightly. At this point, a liquid, which generally comprises the non-volatile material and water, filters through the extraction basket under the increased gas pressure of the chamber, and is removed from the system through the opened ball valve. The sub-critical gas pressurization and subsequent liquid draining are repeated until no additional liquid drains from the system upon opening the ball valve. The SCF chamber is then sealed, and a SCF extraction process is initiated to remove any residual continuous phase, under the following conditions: 90 wt.% CO₂ with 10 wt.% ethanol at a total flow rate of 100 g/min (up-flow), 200 bar, and 35 °C.

In this prophetic example, insulin microspheres, which are substantially free of solvent and non-volatile material are obtained.

### EXAMPLE 4

This prophetic example also discloses a process to remove at least a portion of a non-volatile material and/or a solvent from a dispersion comprising microparticles to thereby harvest microparticles substantially free of non-volatile material and/or solvent in a single process.

A dispersion containing a dispersed phase of microparticles containing plasmid DNA (pCMV, Promega, WI) in a continuous phase comprising a non-volatile material (PEG 3350) and polyvinylpyrrolidone is loaded into a stainless steel extraction basket, and placed in a supercritical fluid vessel chamber. The SCF chamber is then sealed and pressurized to 40 bar with carbon dioxide. The CO₂ supply is turned off to partially depressurize the SCF chamber (the pressure was dropped to about 5 bar), and a ball valve at the bottom of the SCF chamber was opened slightly. At this point, a liquid, which generally comprises the non-volatile material and solvent, filters through the extraction basket under the increased gas pressure of the chamber, and is removed from the system through the opened ball valve. The sub-critical gas pressurization and subsequent liquid draining are repeated until no additional liquid drains from the system upon opening the ball valve. The SCF chamber is then sealed, and a SCF/co-solvent extraction process is initiated to remove any residual continuous phase and/or solvent, under the following conditions: 90 wt.% CO₂ with 10 wt.% ethanol at a total flow rate of 100 g/min (up-flow), 250 bar, and 20 °C.

In this prophetic example, DNA-containing microspheres, which are substantially free of solvent and non-volatile material are obtained.

### EXAMPLE 5

This example demonstrates the successful removal of various non-volatile materials using super-critical or sub-critical fluids.

A sample containing aapproximately twenty grams of one or more liquid non-volatile materials (PEG 200, PEG 300, tetraglycol, propylene glycol, or a combination comprising a 1:1 ratio of propylene glycol to PLURONIC® L10) were each individually loaded into a stainless steel extraction basket, and placed in a supercritical fluid vessel chamber. The SCF chamber was then sealed and a SCF extraction process was initiated to extract the non-volatile material from the basket under the following conditions: 100 wt. % CO₂ at a total flow rate of 50 g/min (up-flow), 250 bar, and 35 °C. The chamber was depressurized at one bar per minute. In each instance, 20 grams of non-volatile material were extracted within an hour.

Specifically, the non-volatile material remaining after 60 minutes of SCF processing was weighed and compared to the amount of polymer initially loaded into the extraction chamber. The following table shows the amount of polymer removal using SCF compared to original polymer weight.

| Table 1 | |
|---|---|
| Non-volatile material | Amount of non-volatile material removed/ starting weight of non-volatile material (g/g) |
| PEG 200 | 20/20 |
| PEG 300 | 20/20 |
| Tetraglycol | 20/20 |
| Propylene Glycol | 22/24 |
| Propylene Glycol/ Pluronic L10 (1:1) | 20/20 |

This example therefore demonstrates the successful removal (and substantially complete removal in nearly each instance) of various non-volatile materials using super-critical or sub-critical fluids.

## Claims

1. A method for processing multi-phasic dispersions comprising:
providing a multi-phasic dispersion including dispersed and continuous phases, the dispersion comprising solid microparticles and at least a non-volatile material,
positioning the dispersion within an extraction basket;
positioning the extraction basket within a chamber capable of being pressurized;
pressurizing the chamber with a first gas to a pressure less than the supercritical pressure of the gas; and,
contacting the dispersion with the first gas, thereby separating at least a portion of the non-volatile material from the dispersion,
wherein the continuous phase is either aqueous or aqueous-miscible, and the non-volatile material comprises a non-ionic aqueous-soluble polymer or a non-ionic aqueous-miscible polymer.

2. The method of claim 1, wherein the continuous phase further comprises water.

3. The method of claim 1 or 2, wherein the chamber is pressurized to a pressure greater than 10 bar.

4. The method of any one of claims 1 to 3, wherein the solid microparticles comprise at least one active agent.

5. The method of claim 4, wherein the active agent is selected from the group consisting of bioactive agents, pharmaceutical agents, diagnostic agents, nutritional supplements, and cosmetic agents.

6. The method of claim 4, wherein the active agent is a bioactive agent comprising at least one bioactive macromolecule selected from the group consisting of carbohydrates, peptides, proteins, vectors, nucleic acids, complexes thereof, conjugates thereof, and combinations thereof.

7. The method of any one of claims 1 to 6, wherein the continuous phase comprises a solution containing at least one of a buffer and a salt.

8. The method of any one of claims 1 to 7, wherein at least the continuous phase comprises the non-volatile material.

9. The method of claim 8, wherein the non-volatile material is selected from the group consisting of non-ionic polyethers, non-ionic copolyethers, non-ionic polyesters, non-ionic copolyesters, non-ionic polyether-polyester copolymers, non-ionic vinyl polymers, non-ionic pyrrolidone-containing polymers, non-ionic polymeric carbohydrates, derivatives and salts of the foregoing materials, and combinations thereof.

10. The method of any one of claims 1 to 9, wherein the first gas is selected from carbon dioxide, nitrogen, compressed air, and mixtures thereof.

11. The method of any one of claims 1 to 10, wherein the first gas is heated to a temperature below the supercritical temperature of the gas.

12. The method of any one of claims 1 to 11, wherein the first gas comprises carbon dioxide and the chamber is pressurized to a pressure between 25 bar and 65 bar.

13. The method of any one of claims 1 to 12, further comprising depressurizing the chamber, and draining a liquid separated from the multi-phasic dispersion.

14. The method of claim 13, further comprising pressurizing the chamber with a second gas to a pressure greater than or equal to the supercritical pressure of the second gas, heating the second gas to provide a supercritical fluid or a sub-critical fluid within the chamber, and contacting the multi-phasic dispersion with the fluid, thereby removing residual non-volatile material and/or solvent from the multi-phasic dispersion to provide microparticles that are substantially free of the non-volatile material and/or the solvent.

15. The method of claim 14, wherein the second gas comprises a gas selected from the group consisting of carbon dioxide, isopropanol, methanol, ethanol, water, toluene, ethylene, xenon, ethane, dimethyl ether, nitrous oxide, propane, ammonia, butane, pentane, and mixtures thereof.

16. The method of claim 15, wherein the second gas comprises carbon dioxide and the chamber is pressurized to a pressure greater than 100 bar.

## Patentansprüche

1. Verfahren zum Verarbeiten von mehrphasigen Dispersionen, umfassend:
das Bereitstellen einer mehrphasigen Dispersion, die dispergierte und zusammenhängende Phasen enthält, wobei die Dispersion feste Mikroteilchen und
mindestens ein nichtflüchtiges Material umfasst;
das Positionieren der Dispersion innerhalb eines Extraktionskorbs;
das Positionieren des Extraktionskorbs innerhalb einer Kammer, die geeignet ist, unter Druck gesetzt zu werden;
das Unterdrucksetzen der Kammer mit einem ersten Gas auf einen Druck geringer als der superkritische Druck des Gases; und
das Inkontaktbringen der Dispersion mit dem ersten Gas, wodurch mindestens ein Teil des nichtflüchtigen Materials von der Dispersion abgetrennt wird,
wobei die zusammenhängende Phase entweder wässrig oder mit Wasser mischbar ist und das nichtflüchtige Material ein nichtionisches, wasserlösliches Polymer oder ein nichtionisches, mit Wasser mischbares Polymer umfasst.

2. Verfahren nach Anspruch 1, wobei die zusammenhängende Phase ferner Wasser umfasst.

3. Verfahren nach Anspruch 1 oder 2, wobei die Kammer auf einen Druck von größer als 10 bar unter Druck gesetzt wird.

4. Verfahren nach einem der Ansprüche 1 bis 3, wobei die festen Mikroteilchen mindestens einen Wirkstoff umfassen.

5. Verfahren nach Anspruch 4, wobei der Wirkstoff aus der Gruppe ausgewählt ist, bestehend aus bioaktiven Mitteln, pharmazeutischen Mitteln, diagnostischen Mitteln, Futtermittelzusatzstoffen und kosmetischen Mitteln.

6. Verfahren nach Anspruch 4, wobei der Wirkstoff ein bioaktives Mittel ist, umfassend mindestens ein bioaktives Makromolekül, ausgewählt aus der Gruppe, bestehend aus Kohlenhydraten, Peptiden, Proteinen, Vektoren, Nukleinsäuren, Komplexen davon, Konjugaten davon und Kombinationen davon.

7. Verfahren nach einem der Ansprüche 1 bis 6, wobei die zusammenhängende Phase eine Lösung, enthaltend mindestens eines von einem Puffer oder einem Salz, umfasst.

8. Verfahren nach einem der Ansprüche 1 bis 7, wobei mindestens die zusammenhängende Phase das nichtflüchtige Material umfasst.

9. Verfahren nach Anspruch 8, wobei das nichtflüchtige Material aus der Gruppe ausgewählt ist, bestehend aus nichtionischen Polyethern, nichtionischen Copolyethern, nichtionischen Polyestern, nichtionischen Copolyestern, nichtionischen Polyether-Polyester Copolymeren, nichtionischen Vinylpolymeren, nichtionischen Pyrrolidon-enthaltenden Polymeren, nichtionischen polymeren Kohlenhydraten, Derivaten und Salzen der vorstehenden Materialien und Kombinationen davon.

10. Verfahren nach einem der Ansprüche 1 bis 9, wobei das erste Gas aus Kohlenstoffdioxid, Stickstoff, komprimierter Luft und Gemischen daraus ausgewählt ist.

11. Verfahren nach einem der Ansprüche 1 bis 10, wobei das erste Gas auf eine Temperatur unterhalb der superkritischen Temperatur des Gases erwärmt wird.

12. Verfahren nach einem der Ansprüche 1 bis 11, wobei das erste Gas Kohlenstoffdioxid umfasst und die Kammer auf einen Druck zwischen 25 bar und 65 bar unter Druck gesetzt wird.

13. Verfahren nach einem der Ansprüche 1 bis 12, weiter umfassend das Herabsetzen des Druckes der Kammer und das Ablassen einer von der mehrphasigen Dispersion abgetrennten Flüssigkeit.

14. Verfahren nach Anspruch 13, weiter umfassend das Unterdrucksetzen der Kammer mit einem zweiten Gas auf einen Druck, der größer oder gleich dem superkritischen Druck des zweiten Gases ist, das Erwärmen des zweiten Gases, um ein superkritisches Fluid oder ein unterkritisches Fluid innerhalb der Kammer bereitzustellen, und das Inkontaktbringen der mehrphasigen Dispersion mit dem Fluid, wodurch restliches nichtflüchtiges Material und/oder Lösungsmittel von der mehrphasigen Dispersion entfernt wird, um Mikroteilchen bereitzustellen, die im Wesentlichen frei von dem nichtflüchtigen Material und/oder dem Lösungsmittel sind.

15. Verfahren nach Anspruch 14, wobei das zweite Gas ein Gas umfasst, ausgewählt aus der Gruppe, bestehend aus Kohlenstoffdioxid, Isopropanol, Methanol, Ethanol, Wasser, Toluol, Ethylen, Xenon, Ethan, Dimethylether, Stickstoffoxid, Propan, Ammoniak, Butan, Pentan und Gemischen daraus.

16. Verfahren nach Anspruch 15, wobei das zweite Gas Kohlenstoffdioxid umfasst und die Kammer auf einen Druck von größer als 100 bar unter Druck gesetzt wird.

## Revendications

1. Procédé de traitement de dispersions à plusieurs phases comprenant :
la préparation d'une dispersion à plusieurs phases comportant des phases dispersées et continues, la dispersion comprenant des microparticules solides et au moins un matériau non volatil,
la mise en place de la dispersion à l'intérieur d'un panier d'extraction ;
la mise en place du panier d'extraction à l'intérieur d'une chambre pouvant être pressurisée ;
la pressurisation de la chambre avec un premier gaz à une pression inférieure à la pression supercritique du gaz ; et
la mise en contact de la dispersion avec le premier gaz, séparant ainsi au moins une partie du matériau non volatile de la dispersion,
dans lequel la phase continue est soit aqueuse soit miscible dans l'eau, et le matériau non volatil comprend un polymère hydrosoluble non ionique ou un polymère miscible dans l'eau non ionique.

2. Procédé selon la revendication 1, dans lequel la phase continue comprend en outre de l'eau.

3. Procédé selon la revendication 1 ou 2, dans lequel la chambre est pressurisée à une pression supérieure à 10 bars.

4. Procédé selon l'une quelconque des revendications 1 à 3, dans lequel les micro-particules solides comprennent au moins un agent actif.

5. Procédé selon la revendication 4, dans lequel l'agent actif est sélectionné à partir du groupe constitué par des agents bio-actifs, des agents pharmaceutiques, des agents de diagnostic, des suppléments nutritionnels, et des agents cosmétiques.

6. Procédé selon la revendication 4, dans lequel l'agent actif est un agent bio-actif comprenant au moins une macromolécule bio-active sélectionnée à partir du groupe constitué par des hydrates de carbone, des peptides, des protéines, des vecteurs, des acides nucléiques, des complexes de ceux-ci, des conjugués de ceux-ci, et des mélanges de ceux-ci.

7. Procédé selon l'une quelconque des revendications 1 à 6, dans lequel la phase continue comprend une solution contenant au moins un tampon et un sel.

8. Procédé selon l'une quelconque des revendications 1 à 7, dans lequel au moins la phase continue comprend le matériau non volatile.

9. Procédé selon la revendication 8, dans lequel le matériau non volatile est sélectionné à partir du groupe constitué par des polyéthers non ioniques, des co-polyéthers non ioniques, des polyesters non ioniques, des co-polyesters non ioniques, des co-polymères polyéther-polyesters non ioniques, des polymères vinyles non ioniques, des polymères contenant de la pyrrolidone non ionique, des polymères d'hydrates de carbone non ioniques, des dérivés et sels des matériaux précédents, et des mélanges de ceux-ci.

10. Procédé selon l'une quelconque des revendications 1 à 9, dans lequel le premier gaz est sélectionné à partir du dioxyde de carbone, de l'azote, de l'air comprimé, et de mélanges de ceux-ci.

11. Procédé selon l'une quelconque des revendications 1 à 10, dans lequel le premier gaz est chauffé à une température supérieure à la température supercritique du gaz.

12. Procédé selon l'une quelconque des revendications 1 à 11, dans lequel le premier gaz comprend du dioxyde de carbone et la chambre est pressurisée à une pression comprise entre 25 bars et 65 bars.

13. Procédé selon l'une quelconque des revendications 1 à 12, comprenant en outre la dépressurisation de la chambre et la purge d'un liquide séparé de la dispersion à plusieurs phases.

14. Procédé selon la revendication 13, comprenant en outre la pressurisation de la chambre avec un second gaz à une pression supérieure ou égale à la pression supercritique du second gaz, le chauffage du second gaz afin de former un fluide supercritique ou un fluide subcritique dans la chambre, et la mise en contact avec la dispersion à plusieurs phases avec le fluide, éliminant ainsi le matériau non volatile résiduel et/ou le solvant de la dispersion à plusieurs phases afin de former des microparticules qui sont sensiblement exemptes du matériau non volatile et/ou du solvant.

15. Procédé selon la revendication 14, dans lequel le second gaz comprend un gaz sélectionné à partir du groupe constitué par le dioxyde de carbone, l'isopropanol, le méthanol, l'éthanol, l'eau, le toluène, l'éthylène, le xénon, l'éthane, l'éther de diméthyle, l'oxyde d'azote, le propane, l'ammoniac, le butane, le pentane, et des mélanges de ceux-ci.

16. Procédé selon la revendication 15, dans lequel le second gaz comprend du dioxyde de carbone et la chambre est pressurisée à une pression supérieure à 100 bars.
